# EUROPEAN PATENT APPLICATION

(11) **EP 4 296 355 A1**
(43) Date of publication of application: **27.12.2023**
(21) Application number: 22756235.2
(22) Date of filing: 16.02.2022
(51) Int. Cl.: C12N 5/10, A61K 35/28, A61K 35/35, A61P 3/04, A61P 9/10, A61P 13/12, A61P 29/00, A61P 31/04, C07K 14/475, C07K 14/50, C12N 5/077, C12N 5/0775, C12N 15/90, C12P 21/02, A01K 67/027

(54) **MESENCHYMAL STEM CELLS AND ADIPOCYTES FOR PREPARING MITOKINE MIXTURE, AND THERAPEUTIC OR PROPHYLACTIC DRUG**

(30) Priority: 17.02.2021 JP 2021023574
(71) Applicant: TOKYO UNIVERSITY OF SCIENCE FOUNDATION, Tokyo 162-8601 (JP)
(72) Inventor: HIGAMI, Yoshikazu, Tokyo 162-8601 (JP); KOBAYASHI, Masaki, Tokyo 162-8601 (JP); NARITA, Takumi, Tokyo 162-8601 (JP); TAKI, Kanari, Tokyo 162-8601 (JP); HIRAO, Yuto, Tokyo 162-8601 (JP)
(74) Representative: Isarpatent
(86) International application number: PCT/JP2022/006200
(87) International publication number: WO 2022/176917

(57) **Abstract**

Provided are: a non-human animal or a part of the same in which the function of Mipep gene is totally or partially lost in adipose tissues or the expression level of Mipep gene in adipose tissues is lowered compared to a wild type; and mesenchymal stem cells or adipocytes in which the function of Mipep gene is totally or partially lost or the expression level of Mipep gene is lowered compared to a wild type. Also provided are: a therapeutic or prophylactic drug that comprises the aforesaid mesenchymal stem cells or adipocytes or a culture supernatant thereof; and a method for preparing a mitokine mixture using the aforesaid non-human cells, mesenchymal stem cells or adipocytes.

## Description

### TECHNICAL FIELD

The present invention relates to mesenchymal stem cells and adipocytes for preparing a mitokine mixture and a therapeutic or prophylactic drug.

### BACKGROUND ART

A mitochondrial unfolded protein response (mtUPR) is a (stress) response induced for maintaining mitochondrial proteostasis in response to mitochondrial stress. In this response, groups of genes such as chaperone, protease and mitokine genes are involved.

Typical genes included in a mitokine gene group known in the art are GDF15 (growth/differentiation factor 15) and FGF21 (fibroblast growth factor 21). Of them, GDF15 is a member belonging to the transforming growth factor beta superfamily and dominantly expressed in the liver, lung and kidney of healthy animals, and expressed in various tissues in response to different types of stresses. It has been suggested that GDF15 may be involved in resistance against diseases, for example, bacterial and viral infections and sepsis (see Non-Patent Document 1). GDF15 is induced in the heart. Based on this, it has been clarified that GDF15 exhibits a defensive function against ischemia/reperfusion injury (see, Non-Patent Document 2).

In contrast, FGF21 is a member belonging to the endocrine system FGF superfamily. FGF21 was initially identified as a hepatokine but has recently been reported to be expressed in white adipose tissues, brown adipose tissues and other tissues such as muscle or pancreatic tissues. It has been confirmed that FGF21 is also involved in stress and reported that administration of FGF21 to, for example, diabetic mice or aged mice, inhibits neuron loss, increases the production of antioxidant enzymes and enhances the protective effect on mitochondria in neurons (see, Non-Patent Document 3). It has been also reported that the administration of FGF21 regulates the expression of galectin-3. Thus, FGF21 may be used as a therapy for hypoxia-induced heart injury (see, Non-Patent Document 4).

As mentioned above, it has been suggested that a gene included in a mitokine gene group may be involved in therapy or prophylaxis for various diseases. Because of this, it is expected to efficiently prepare the product of a gene included in a group of mitokine genes.

Non-Patent Document 1: Luan et al., Cell, 2019, 178, pp. 1231-1244
Non-Patent Document 2: Kempf et al., Circ. Res., 2006, 98, pp. 351-360
Non-Patent Document 3: Kang et al., Biomed. Pharmacother., 2020, 129, 110439
Non-Patent Document 4: Sun et al., J. Cell Biochem., 2019, 120(12), pp. 19529-19540

### DISCLOSURE OF THE INVENTION

### Problems to be Solved by the Invention

The present invention was proposed in consideration of these circumstances. An object of the invention is to efficiently prepare the product of a gene included in a group of mitokine genes.

### Means for Solving the Problems

The present inventors conducted intensive studies on the object. As a result, they found that the expression of a gene included in a mitokine gene group is increased by impairing the function of a Mipep gene in the adipose tissues. Based on the finding, they arrived at the completion of the present invention. More specifically, the present invention provides the followings.

<1> A non-human animal or a part thereof having a totally or partially lost function of a Mipep gene in an adipose tissue or a lowered expression level of the Mipep gene in an adipose tissue compared to a wild type thereof.
<2> Mesenchymal stem cells having a totally or partially lost function of a Mipep gene or a lowered expression level of the Mipep gene compared to a wild type thereof.
<3> Adipocytes having a totally or partially lost function of a Mipep gene or a lowered expression level of the Mipep gene compared to a wild type thereof.
<4> A therapeutic or prophylactic drug containing at least one selected from the group consisting of mesenchymal stem cells or adipocytes having a totally or partially lost function of a Mipep gene or a lowered expression level of the Mipep gene compared to a wild type thereof, and a culture supernatant of the mesenchymal stem cells or adipocytes containing a mitokine.
<5> The therapeutic or prophylactic drug as described in aspect <4>, in which the therapeutic or prophylactic drug is used for treating or preventing at least one selected from the group consisting of sepsis, ischemia/reperfusion injury, an inflammatory disease, a chronic kidney disease, obesity, atherosclerosis, a nonalcoholic fatty liver disease and other metabolic diseases.
<6> A method for preparing a mitokine mixture, including collecting a plurality of types of mitokines from at least one selected from the group consisting of a non-human animal having a totally or partially lost function of a Mipep gene in an adipose tissue or a lowered expression level of the Mipep gene compared to a wild type thereof, and mesenchymal stem cells or adipocytes having a totally or partially lost function of the Mipep gene or a lowered expression level of the Mipep gene compared to a wild type thereof.

### Effects of the Invention

According to the present invention, it is possible to efficiently prepare the product of a gene included in a group of mitokine genes.

### BRIEF DESCRIPTION OF THE DRAWINGS

FIG. 1 shows the expression levels of the Mipep gene.
FIG. 2 shows changes of Mipep substrate proteins by a Mipep gene defect.
FIG. 3A shows a change of an adipose tissue by a Mipep gene defect.
FIG. 3B shows changes of adipose tissues by a Mipep gene defect.
FIG. 4A shows the visceral fat levels by a Mipep gene defect.
FIG. 4B shows the visceral fat levels by a Mipep gene defect.
FIG. 5 shows changes in gene expressions involved in mtUPR.
FIG. 6 shows the effect of a Mipep gene defect on plasma GDF15 level.
FIG. 7 shows an effect of a Mipep gene defect on resistance against the administration of LPS.

### PREFERRED MODE FOR CARRYING OUT THE INVENTION

Now, embodiments for carrying out the present invention will be more specifically described. However, the present invention is not limited to the following embodiments.

The term "mitokine" as used herein is sometimes pronounced as "maito-kine". A mitokine is a physiologically active substance that extracellularly transmits stress generated in mitochondria and exerts a non-cell autonomous function.

### [Non-human animal or a part thereof]

In the non-human animal according to the embodiment, the function of the Mipep gene is totally or partially impaired in adipose tissues or the expression level of the Mipep gene in adipose tissues is low compared to a wild type thereof. As described later in Examples, if the Mipep gene is deleted in adipose tissues, the expression of a gene included in a mitokine gene group increases.

MIPEP (mitochondrial intermediate peptidase) is MtSPase, which plays a role in second-time processing of a substrate protein processed (cut) by mitochondrial processing peptidase (MPP) and known to be an enzyme that cleaves an octapeptide of the protein cut by MPP, from the N terminal. Note that, the present inventors previously conducted global gene expression analysis and reported that calorie restriction, which acts to improve metabolism, prevent aging and extends lifetime, accelerates expression of MIPEP, and that SIRT3, which is a deacetylase localized in mitochondria and involved in activation of a plurality of mitochondria-localized enzymes, is a substrate of MIPEP (Kobayashi et al., FEBS Letters, 2017, 591, 4067-4073).

Information on the nucleotide sequence and amino acid sequence of the Mipep gene is available from the GenBank database of the National Center For Biotechnology Information (NCBI). For example, the nucleotide sequence and amino acid sequence of human Mipep are represented by SEQ ID NOs: 1 and 2, respectively.

A non-human animal having a totally or partially lost function of Mipep gene refers to an animal in which the Mipep gene is destroyed so as not to function or modified by recombination. In the Mipep gene, one of the alleles on the genome may be destroyed or mutated so as not to function or both of the alleles may be destroyed or mutated. The offspring of such an animal is included in the non-human animal.

The phrase "totally lost function of Mipep gene" means that the Mipep gene is completely impaired. In contrast, the phrase "partially lost function of Mipep gene" means that a part of the Mipep gene is impaired, with the result that the function of the Mipep gene is lowered compared to the function of a wild type thereof. If such a condition is satisfied, the Mipep gene is not expressed at all or even if expressed, the activity of the protein is lowered or impaired.

The non-human animal having a totally or partially lost function of Mipep gene can be prepared by a method commonly known in the art. A standard method thereof indludes the following steps (a) to (d):
(a) preparing ES cells having a totally or partially lost function of Mipep gene.
(b) transplanting the ES cells into the embryo of a non-human animal and allowing the animal to give birth to prepare a chimeric non-human animal.
(c) mating the chimeric non-human animal with an allogeneic wild-type non-human animal to prepare a hetero-knockout non-human animal.
(d) mutually mating hetero-knockout non-human animals to prepare a homo-knockout non-human animal.

The phrase "lowered expression level of Mipep gene compared to a wild type thereof" means that the expression level of the Mipep gene is suppressed and lowered compared to the expression level of a wild type thereof. The expression level of the Mipep gene can be suppressed, for example, by reducing the amount of transcription of the Mipep gene or inhibiting translation thereof.

Examples of the method for suppressing the expression level of the Mipep gene include, but are not particularly limited to, RNA interference (RNAi). RNAi may be induced by designing and synthesizing, for example, siRNA (small interfering RNA), shRNA (short hairpin RNA) or miRNA (micro RNA) for the Mipep gene, integrating such RNA into a retroviral vector or an adenovirus vector, and then, introducing the vector into a non-human animal.

The non-human animal is not particularly limited as long as it is an animal except a human. Examples of the non-human animal include a mouse, rat, guinea pig, hamster, rabbit, monkey, cow, mini pig, pig, sheep, goat, dog and cat. Of these, it is preferable that examples of the non-human animal include a mouse or a rat in consideration that a protocol for preparing a mutant animal has been established and reproduction is easy.

The part of a non-human animal is not particularly limited as long as it can be taken from the non-human animal. Examples of the part of a non-human animal include a tissue, cell, debris or extract of these and body fluid derived from the non-human animal.

### [Mesenchymal stem cells]

The mesenchymal stem cells according to the embodiment have a totally or partially lost function of the Mipep gene or a lowered expression level of the Mipep gene compared to a wild type thereof. It has been reported that a gene included in a mitokine gene group is expressed in the mesenchymal stem cells (see, for example, International Publication No. WO2017/188403). Similarly in adipocytes (described later), production of a mitokine is enhanced by impairing the function of the Mipep gene. Because of this, the mesenchymal stem cells can be used, for example, in cell therapy, a therapy in which the cells are directly transplanted. In the body in which the cells are transplanted, a larger amount of mitokine can be produced.

If mesenchymal stem cells are cultured, the culture supernatant can be collected. As mentioned above, since the mesenchymal stem cells produce a mitokine, the culture supernatant contains the mitokine. Thus, if the culture supernatant of mesenchymal stem cells is used as a therapeutic or prophylactic drug, it may be expected to produce a therapeutic or prophylactic effect thereof on diseases for which a mitokine effectively works. Since the culture supernatant contains a plurality of types of mitokines with balance as well as other useful components, another effect is expected compared to the case of directly administering mitokines.

The phrases "totally lost function of Mipep gene", "partially lost function of Mipep gene" and "lowered expression level of Mipep gene compared to a wild type thereof" are the same as specified in the above section [Non-human animal or a part thereof].

The mesenchymal stem cells are derived from the mesenchyme. As long as the mesenchymal stem cells are somatic stem cells having the self-replicating ability and differentiation ability, the tissue of origin (from which the mesenchymal stem cells are derived) is not particularly limited. Examples of the tissue of origin include the adipose, bone marrow, pulp, blood (e.g., peripheral blood, cord blood), placenta, umbilical cord, synovium, periosteum, perichondrium, muscle, ligament, tendon, meniscus and skin tissues. Of them, the adipose tissue is preferred as the tissue of origin.

The mesenchymal stem cells may be cells derived from a non-human animal as mentioned above or a human.

The mesenchymal stem cells may be derived from the cells (autologous cells) of the individual, i.e., the subject to which the mesenchymal stem cells to be administered or the cells (allogeneic cells) of an individual except the subject.

The mesenchymal stem cells may be, e.g., cells derived from ES cells by differentiation induction, cells derived from induced pluripotent stem cells (e.g., iPS cells) by differentiation induction, established cells or Muse cells (Multi-lineage differentiating Stress Enduring Cells).

As the mesenchymal stem cells, cells negative to a differentiation marker (e.g., CD24) and maintaining an undifferentiated state are ordinarily used.

A method for preparing mesenchymal stem cells is not particularly limited. Any method can be employed as long as it is known in the art as a method for preparing mesenchymal stem cells. For example, there is a method including seeding cells, which contain mesenchymal stem cells and are obtained from a tissue of origin as mentioned above, in a culture plate, allowing the cells to proliferate while adhering them to the plate, and culturing part of the proliferation cells in a culture plate to proliferate them again.

A method for totally or partially impairing the function of the Mipep gene in mesenchymal stem cells is not limited; for example, a method using genome editing by, e.g., CRISPR/Cas nuclease, can be used. The method for suppressing the expression level of the Mipep gene in the mesenchymal stem cells is not particularly limited; for example, a method using RNAi as mentioned above for the non-human animal can be used.

### [Adipocytes]

The adipocytes according to the embodiment have a totally or partially lost function of the Mipep gene or a lowered expression level of the Mipep gene compared to a wild type thereof. As shown in Examples (later described), a large amount of mitokine is produced in such adipocytes. Because of this, the adipocytes can be used, for example, in a cell therapy, a therapy in which the cells are directly transplanted. In the body in which the cells are transplanted, a larger amount of mitokine can be produced.

If adipocytes are cultured, the culture supernatant can be collected. Since a large amount of mitokine is produced in the adipocytes as mentioned above, the culture supernatant thereof contains a large amount of mitokine. Thus, if the culture supernatant of adipocytes is used as a therapeutic or prophylactic drug, it may be expected to produce a therapeutic or prophylactic effect thereof on diseases for which a mitokine effectively works. Since the culture supernatant contains a plurality of types of mitokines with balance as well as other useful components, another effect is expected compared to the case of directly administering mitokines.

The phrases "totally lost function of Mipep gene", "partially lost function of Mipep gene" and "lowered expression level of Mipep gene compared to a wild type thereof" are as specified above for the non-human animal.

The adipocytes may be the cells separated from a non-human animal or the cells obtained by differentiation of mesenchymal stem cells as mentioned above.

A method for differentiating adipocytes from mesenchymal stem cells is not particularly limited; for example, a method of bringing, e.g., dexamethasone, insulin, 3-isobutyl-1-methylxanthine, rosiglitazone, glucocorticoid or a phosphodiesterase inhibitor into contact with mesenchymal stem cells, is known. Alternatively, an agent commercially available, for example, as a "differentiation inducer", can be used.

The adipocytes may be the cells derived from a non-human animal as mentioned above or a human.

The adipocytes may be derived from the cells (autologous cells) of the individual, i.e., the subject to which the mesenchymal stem cells to be administered or the cells (allogeneic cells) of an individual except the subject.

A method for totally or partially impairing the function of the Mipep gene in adipocytes is not limited; for example, a method such as genome editing using, e.g., CRISPR/Cas nuclease can be used. The method for suppressing the expression level of the Mipep gene in the adipocytes is not particularly limited; for example, a method using RNAi as mentioned for the non-human animal can be used.

### [Therapeutic or prophylactic drug]

The therapeutic or prophylactic drug contains at least one selected from the group consisting of mesenchymal stem cells or adipocytes having a totally or partially lost function of the Mipep gene or a lowered expression level of the Mipep gene compared to a wild type thereof, and a culture supernatant of the mesenchymal stem cells or adipocytes containing a mitokine.

The mesenchymal stem cells or adipocytes that can be employed are the same cells as mentioned in the above sections: [Mesenchymal stem cells] and [Adipocytes].

The culture supernatant of the mesenchymal stem cells or adipocytes containing a mitokine is obtained during a process for culturing mesenchymal stem cells or adipocytes having a totally or partially lost function of the Mipep gene or a lowered expression level of the Mipep gene compared to a wild type thereof. The method for obtaining the culture supernatant is not particularly limited. Now, an example of the method will be described.

First, from a non-human animal having a totally or partially lost function of the Mipep gene in an adipose tissue or a lowered expression level of the Mipep gene compared to a wild type thereof, the adipose tissue is subcutaneously taken. The adipose tissue, if necessary, treated with, e.g., collagenase, was cultured in the presence of an appropriate culture solution. The culture supernatant is collected at a rate of once per several days after initiation of culture. The culture supernatant collected is subjected to an appropriate sterilization treatment (preferably, sterilization without heating such as filter filtration or UV sterilization), and then, the residual virus is preferably checked.

The culture supernatant of the mesenchymal stem cells or adipocytes thus prepared contains a large amount of mitokine. Examples of the mitokines include, but are not particularly limited to, GDF15, FGF21 and ANGPTL6.

The therapeutic or prophylactic drug is preferably used for a disease for which a gene included in a mitokine gene group effectively works. Examples of the disease include sepsis, ischemia/reperfusion injury, an inflammatory disease, a chronic kidney disease, obesity, atherosclerosis, a nonalcoholic fatty liver disease and other metabolic diseases.

The therapeutic or prophylactic drug may further contain pharmaceutically acceptable additives (pharmaceutical additives) as long as they do not impair the function of the drug, in addition to the mesenchymal stem cells, adipocytes or culture supernatant. Examples of the additives include, but are not limited to, a stabilizer, preservative, buffer, pH regulator, suspending agent, fragrance, coloring agent and thickening agent. The therapeutic or prophylactic drug may contain components derived from a culture medium.

The therapeutic or prophylactic drug may be mesenchymal stem cells, adipocytes or culture supernatant, which are/is directly contained in a solvent, contained in the form of, e.g., sheet, tube or layer, or immobilized form on a solid phase.

In the case where adipocytes are used as the therapeutic or prophylactic drug, the dosage form (transplantation method) for adipocytes to a human is prepared by adding adipocytes in saline or a culture solution so as to have a density of, for example, 1 × 10³ to 1 × 10⁷ cells/mL and locally injected or administered intraperitoneally or subcutaneously by use of, e.g., a catheter. Examples of the other dosage form of the therapeutic or prophylactic drug include intravenous administration, intra-arterial administration, intramuscular administration, intranasal administration, spinal intraluminal implantation, intra-articular implantation and gum injection.

An application target for the therapeutic or prophylactic drug is not particularly limited, but preferably is, e.g., a mammal. The mammal may be either a human or a non-human animal.

The dose of the therapeutic or prophylactic drug is appropriately determined depending on, e.g., the administration target, administration route, target disease and/or symptoms.

The therapeutic or prophylactic drug may be administered in combination with another agent depending on the administration purpose. The type and amount of the agent to be administered in combination with the therapeutic or prophylactic drug are appropriately selected depending on the effect to be desired. The drug may be administered together with or separately from the therapeutic or prophylactic drug.

### [Method for preparing mitokine mixture]

As shown in Examples (later described), the expression levels of a plurality of genes contained in a mitokine gene group are increased by impairing the function of the Mipep gene in the adipose tissues. Accordingly, a mitokine mixture can be prepared by collecting a plurality of types of mitokines from at least one selected from the group consisting of a non-human animal having a totally or partially lost function of the Mipep gene in adipose tissues or a lowered expression level of the Mipep gene compared to a wild type thereof, and mesenchymal stem cells or adipocytes having a totally or partially lost function of the Mipep gene or a lowered expression level of the Mipep gene compared to a wild type thereof.

Examples of the mitokine to be contained in the mitokine mixture prepared are the same as specified in the above section [Therapeutic or prophylactic drug].

### EXAMPLES

Now, the present invention will be described more specifically by way of Examples, but the invention is not limited to these Examples.

### [Preparation of knockout mouse]

Mipep gene knockout mice were prepared by the following method.

### <Preparation of targeting vector and introduction into ES cells>

The targeting vector was prepared by inserting a targeting region of a mouse genome into a DT-A/conditional KO FW vector. An insert (5' arm, targeting arm, 3' arm) was amplified using clone RP23-142O16 (Advanced Geno Techs) derided from a mouse BAC (Bacterial Artificial Chromosome) library as a template and the primers listed in Table 1 by means of KOD FX Neo (TOYOBO Co., Ltd.). Then, the 5' arm thereof was treated with AscI and NotI, the targeting arm was treated with PmeI and SacII, and the 3' arm was treated with SwaI and XhoI, and then, ligated to the DT-A/conditional KO FW vector treated with the same restriction enzymes. Thereafter, the targeting vector was purified. The targeting vector was linearized with treatment with XhoI, and thereafter, introduced into ES cells derived from C57BL/6N by electroporation. The primers used herein are as follows.

**[Table 1]**

| Name of primer | Primer sequence | SEQ ID NO: |
|---|---|---|
| short arm (5' arm) | Forward 5'-GTCGAGGCGCGCCGTAAGCCTCTCCCTGCTG-3' | 3 |
| | Reverse 5'-GACGTGCGGCCGCGTAGCAGAACAGCATCCAC-3' | 4 |
| medium arm (targeting arm) | Forward 5'-AGCTCGTTTAAACCAGTTAGCTCACAGCATG-3' | 5 |
| | Reverse 5'-CGGATCCGCGGGTACAGCCAGGAACCATG-3' | 6 |
| long arm (3' arm) | Forward 5'-GGCCGATTTAAATCTTCTTCTGAAGTCCTCAGTTC-3' | 7 |
| | Reverse 5'-CGATCCTCGAGAGTTACCACTAACATACAGGTAAGG-3' | 8 |
| Southern blotting probe | Forward 5'-ATGATCTCCAGTCTCCAAACAC-3' | 9 |
| | Reverse 5'-TCAGCAGTCCCTTATCCCTTG-3' | 10 |
| Mipep flox genotyping | Forward 5'-GGCTGCTTCCTTCTACAAA-3' | 11 |
| | Reverse 5'-CCTCCTGGTGATGCTCTTTTG-3' | 12 |
| Mipep KO genotyping | Forward 5'-TTTGCCTGGTTCATCTGTGG-3' | 13 |
| | Reverse 5'-CCTCCTGGTGATGCTCTTTTG-3' | 14 |

### <Southern blotting>

Using RP23-142O16 as a template and the primers listed in Table 1, a 3' probe was amplified by KOD FX Neo. Thereafter, genomic DNA was extracted from the ES cells having the targeting vector introduced therein by a phenol/chloroform method and treated with ApaLI. Subsequently, genome DNA (20 µg) treated with ApaLI was applied to 0.80% agarose gel, electrophoresed, stained with ethidium bromide and photographed. Thereafter, the gel was sequentially treated with an acid (soaked in 0.25 M HCl at room temperature for 15 min) and with a base (soaked in 0.50 M NaOH and 1.5 M NaCl at room temperature for 15 min) and neutralized (soaked in 0.50 M Tris-HCl (pH8.0) and 1.5 M NaCl at room temperature for 20 min) in this order. After treatment, the DNA was transferred on a nylon membrane (Gene Screen Plus) by use of osmotic pressure of 10 × SSC (1.5 M NaCl and 150 mM sodium citrate) and allowed to cross-link at UV (150 mJ). After the crosslinking, the nylon membrane was soaked in a buffer containing 5 × SSCP (0.75 M NaCl, 75 mM sodium citrate, 50 mM NaH₂PO₄, 5.0 mM EDTA), 50% Formamide, 2 × Denhardt's solution, 1.0% SDS and 100 ug/mL salmon testis DNA; and prehybridization was carried out at 42°C overnight. Then, a ³²P-labeled 3' probe was added to the buffer and hybridization was further carried out at 42°C overnight. Thereafter, the buffer was removed and the membrane was washed in a 2 × SSC/0.10% SDS solution at 42°C for 15 min, and then, twice in a 0.1 × SSC/0.10% SDS solution at 65°C for 15 min. The membrane was placed on a photosensitive plate and allowed to leave overnight, and thereafter, photographed by FLA-7000 (FUJIFILM).

### <Preparation of mice>

The ES cells, which were confirmed to have a target allele inserted in a desired site by southern blotting, were mixed with a fertilized egg of an ICR mouse in an 8-cell stage and cultured overnight. The fertilized egg in the blastocyst stage was transplanted into the uterus of a pseudo-pregnant mouse to prepare a chimeric mouse (aggregation method). The chimeric mouse was mated with C57BL/6JJcl to obtain a C57BL/6J mouse having the genome derived from the ES cells having the insert. CAG-FLPe mice (Kanki et al., Exp. Anim., 2006, 55, 137-141) was mated with the mouse obtained above. Then, a neomycin-resistant sequence remaining in the ES cell-derived genome was removed by the flippase-FRT system to prepare Mipep^{flox/flox} mice. Thereafter, the Mipep^{flox/flox} mice were mated with Adiponectin-Cre mice to prepare adipose tissue-specific Mipep KO mice.

### [Test Example 1]

In Test Example 1, the expression level of the Mipep gene in the Mipep knockout mice (also referred to as "Mipep KO mouse" herein) was checked by real-time PCR. The real-time PCR was carried out in accordance with the following manner.

First, mRNA was extracted from an adipose tissue by ISOGENII (Nippon gene) and the concentration thereof was checked by a spectrophotometer, NanoDrop 1000 (Thermo Fisher Scientific). Total RNA was subjected to a reverse transcription reaction using ReverTra Ace qPCR RT Master Mix (TOYOBO Co., Ltd.) to obtain cDNA. Thereafter, Real-time RT-PCR using THUNDERBIRD SYBR qPCR Mix (TOYOBO Co., Ltd.) was carried out by the CFX connect real-time PCR system (Bio-rad). Note that, individual reaction conditions were set in accordance with the protocol recommended by the reagent manufacturers. The sequences of the primers used herein are as follows:

**[Table 2]**

| Name of gene | Primer sequence | SEQ ID NO: |
|---|---|---|
| Mipep | Forward 5'-CAAAGGAGAGGTGTGGTGTAATG-3' | 15 |
| | Reverse 5'-GGAAGATTCAGCATGAGAACGAC-3' | 16 |
| Rps18 | Forward 5'-TGCGAGTACTCAACACCAACAT-3' | 17 |
| | Reverse 5'-CTTTCCTCAACACCACATGAGC-3' | 18 |

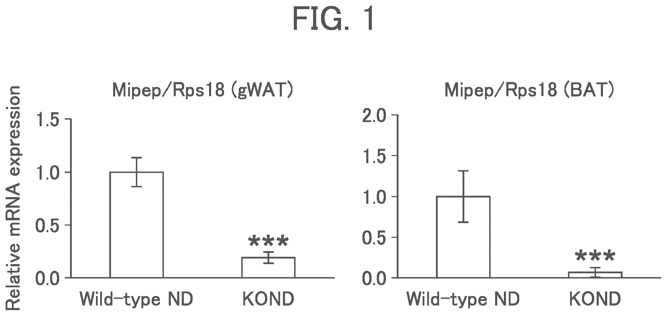

As shown in FIG. 1, it was confirmed that the expression level of the Mipep gene decreases in the adipose tissues of Mipep KO mice.

### [Test Example 2]

In Test Example 2, a change in MIPEP substrate in an adipose tissue by knock-out of Mipep was checked by western blotting. The western blotting was carried out in accordance with the following manner.

To the tissue, an appropriate amount of an SDS sample buffer (50 mM Tris-HCl (pH6.8), 2% SDS, 3 M urea and 6% glycerol) was added. The mixture was homogenized and further sonicated. The lysate thus obtained was centrifuged (12000 × G, 4°C, 30 min). The supernatant was collected and incubated at 95°C for 5 minutes. The amount of protein contained in the supernatant obtained was analyzed by the BCA Protein Assay Kit (Thermo Fisher Scientific). The concentration thereof was adjusted to be 1 mg/mL with the SDS sample buffer. A mixed solution of 0.25% BPB/5% 2-mercaptoethanol = 1:1 was added in a volume of 1/10 of the sample and the mixture was subjected to a reduction treatment (at 95°C for 5 minutes). The sample prepared was electrophoresed by the SDS-PAGE method and transferred to a nitrocellulose membrane. After the transfer, the membrane was shaken in a blocking solution (2.5% skim milk (WAKO)/0.25% BSA in TTBS (25 mM Tris-HCl (pH7.4), 140 mM NaCl, 2.5 mM KCl, 0.1% Tween 20) = 1:1) at room temperature for one hour. A primary antibody was then added to a reaction solution (Immno Shot Reagent I (Cosmo Bio, IS-001)/blocking solution = 2:1) and allowed to react to the membrane at 4°C, overnight or two nights. After completion of the reaction with the primary antibody, the membrane was washed with TTBS (5 minutes × twice and 10 minutes × twice). Then, a secondary antibody was added to a reaction solution (Immuno Shot Reagent II (Cosmo Bio, IS-002)/blocking solution = 2:1). The reaction solution was allowed to react to the membrane for one hour at room temperature. After completion of the reaction with the secondary antibody, the membrane was washed with TTBS (5 minutes × twice and 10 minutes × twice). Subsequently, a chemiluminescence using immunoStar LD (WAKO) was carried out and a picture was taken by the LAS-3000 lumino-image analyzer. Quantification was carried out by Multi Gauge 3.1. Note that, as the primary antibody, an anti-SIRT3 antibody (Cell Signaling, #5490), an anti-COX4 antibody (Cell Signaling, #4844), an anti-MDH2 antibody (Cell Signaling# 8610), an anti-Clpx antibody (Abcam, ab168338), an anti-SPG7 antibody (Thermo Fisher Scientific, PA5-87106) or an anti-MRPL32 antibody (Thermo Fisher Scientific, PA5-109980) was used. As the secondary antibody, a HRP-labeled anti-rabbit IgG antibody (West Grove) was used.

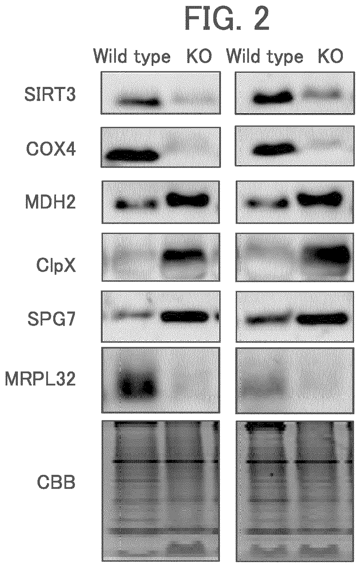

As shown in FIG. 2, in a Mipep KO mouse, the bands of SIRT3 and COX4 proteins serving as substrates of MIPEP shifted upward and the expression levels thereof decreased. Whereas, the band of MDH2 as a substate of MIPEP, similarly shifted upward in a Mipep KO mouse but the expression level of the protein increased. It was considered that the shift of these bands upward may be caused by a failure in maturation. Also as shown in FIG. 2 in a Mipep KO mouse, the expression level of Clpx (a subunit of protease ClpXP) and the expression level of SPG7 (a subunit of protease m-AAA) increased but the expression level of MRPL32 (a substrate of m-AAA) decreased. From the results, it is presumed that MIPEP uses a protease as a substrate and plays an important role for mitochondrial proteostasis.

### [Test Example 3]

In Test Example 3, a change of an adipose tissue by knock-out of Mipep was checked. First, the state of an adipose tissue was checked, and then, the adipose tissue was fixed with a 10% neutral buffered formalin solution (10% formaldehyde in PBS), embedded in paraffin, sectioned into thin slices of 5 µm in thickness and stained with Hematoxylin-Eosin (HE).

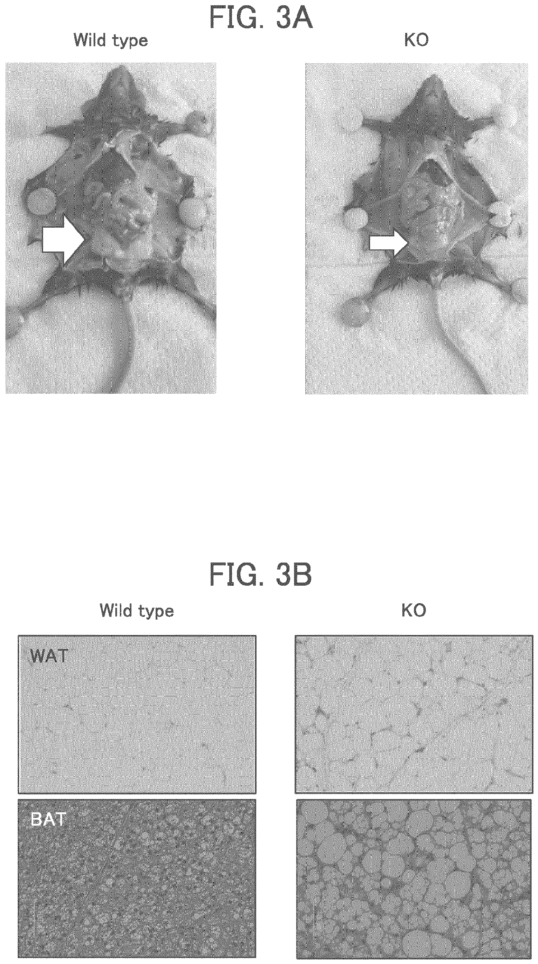

First, as shown in FIG. 3A, it was confirmed that the size of the adipose tissue significantly reduces in a Mipep KO mouse.

Then, as shown in FIG. 3B, in the WAT of a Mipep KO mouse, lipoblast-like cells representing abnormally maturated/undifferentiated adipocytes were observed at several sites. In the BAT of a Mipep KO mouse, it was confirmed that the number of lipid droplets increased.

### [Test Example 4]

In Test Example 4, a change in the amount of visceral fat in the adipose tissue around a mouse genital organ by knock-out of Mipep was checked. A change was checked by subjecting 19 to 20 weeks-old mice to computed tomography as follows. Tomography was carried out by use of a third-generation CT scanner, Latheta LCT-200 (Hitachi-Alola) in the constant conditions of a tube voltage of 50 kV and a current of 0.5 mA. A mouse was placed in a holder having a diameter of 48 mm. Data were collected by rotating a scanning machine 360 degrees. The mouse was photographed at a resolution of 96 µm per pixel, with a width of 192 µm per picture and at intervals of 600 µm. The density within the range of -550 to -140 HU was determined as WAT and subjected to evaluation/analysis. Pictures were taken while keeping an estimated X-ray exposure per mouse to fall within the range of less than 40 mSv.

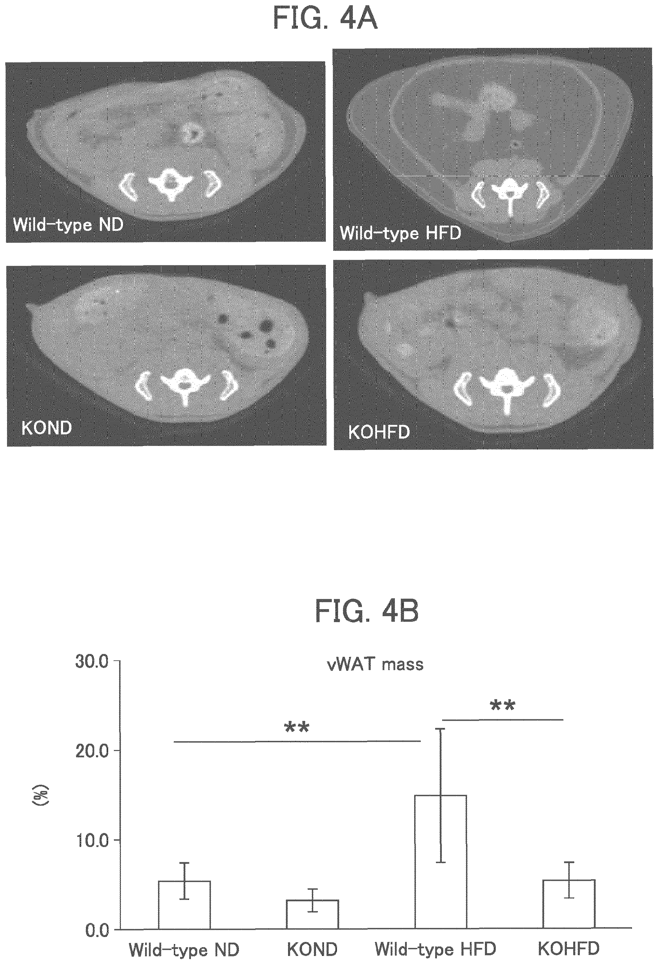

As shown in FIG. 4A and FIG. 4B, the level of visceral fat (vWAT) increases in wild-type mice when a high-fat diet (HFD) was fed; whereas, such an increase was not observed in Mipep KO mice.

### [Test Example 5]

In Test Example 5, a change in mtUPR-associated gene expression in adipose tissues by knock-out of Mipep was checked. The change was checked by real-time PCR in the same manner as in Test Example 1. The sequences of the primers used herein are as follows.

**[Table 3]**

| Name of gene | Primer sequence | SEQ ID NO: |
|---|---|---|
| Adiponectin | Forward 5'-TGCCGAAGATGACGTTACTACAAC-3' | 19 |
| | Reverse 5'-CTTCAGCTCCTGTCATTCCAAC-3' | 20 |
| Leptin | Forward 5'-CCAGGATCAATGACATTTCACACAC-3' | 21 |
| | Reverse 5'-CAGGGAGCAGCTCTTGGAGAAG-3' | 22 |
| PPARγ | Forward 5'-CACAATGCCATCAGGTTTGG-3' | 23 |
| | Reverse 5'-GCGGGAAGGACTTTATGTATGAG-3' | 24 |
| Gdf15 | Forward 5'-AGTGTCCCCACCTGTATCG-3' | 25 |
| | Reverse 5'-TGTCCTGTGCATAAGAACCA-3' | 26 |
| Fgf21 | Forward 5'-GAAGCCCACCTGGAGATCAG-3' | 27 |
| | Reverse 5'-CAAAGTGAGGCGATCCATAGAG-3' | 28 |
| Angptl6 | Forward 5'-ACTACGACAGCTTCTCCTTG-3' | 29 |
| | Reverse 5'-AGTGCTGAAAGGTTTGTCAT-3' | 30 |
| mtHSP70 | Forward 5'-TGGCTATTACTGCGGGTTCT-3' | 31 |
| | Reverse 5'-CATCTGCTCCACCTCCTCT-3' | 32 |
| Hsp60 | Forward 5'-AAATTGCACAGGTTGCTACG-3' | 33 |
| | Reverse 5'-TGATGACACCCTTTCTTCCA-3' | 34 |
| Hsp10 | Forward 5'-AGTGCTGCCGAAACTGTAACC-3' | 35 |
| | Reverse 5'-TCTTTCCTTTCCCTCCTGACC-3' | 36 |
| Clpx | Forward 5'-CAAAAGACGGGGCAAACAAG-3' | 37 |
| | Reverse 5'-AACGCGTGGAAGACACAAAG-3' | 38 |
| Clpp | Forward 5'-CATTCACTGCCCAATTCCAG-3' | 39 |
| | Reverse 5'-TGGTGTGTTTGGCGGCGTAGATG-3' | 40 |
| Parl | Forward 5'-GTTGCCACAGGAAGATATGGAC-3' | 41 |
| | Reverse 5'-GAAGGTGAAGACAGGGAGGAAG-3' | 42 |

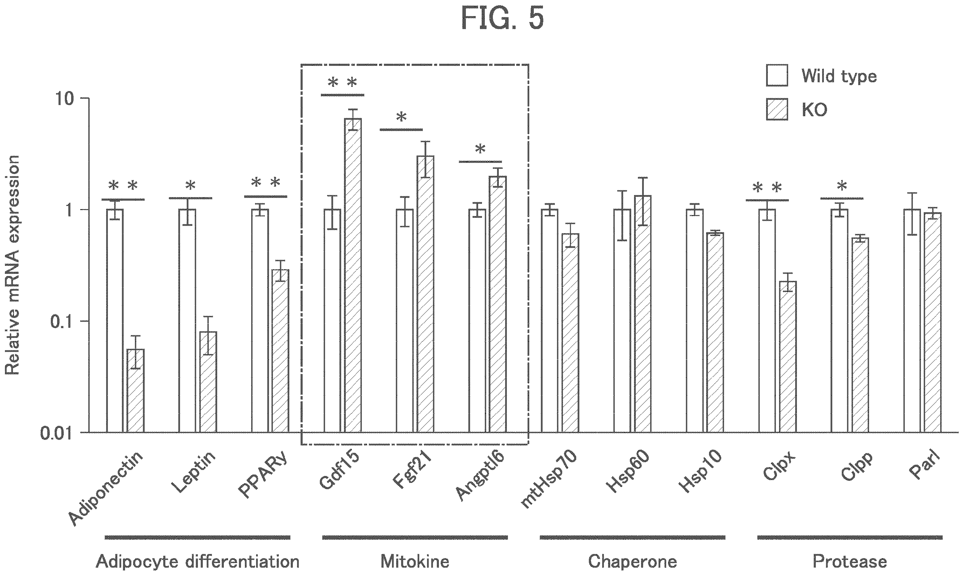

As shown in FIG. 5, the expression levels of genes included in a mitokine gene group increased in Mipep KO mice compared to wild-type mice. In contrast, the expression levels of the genes included in groups of chaperone and protease genes did not increase. It was confirmed that the expression levels of the genes involved in the differentiation of adipocytes are significantly low in Mipep KO mice.

Note that, when gene expression was globally analyzed by use of an RNA-seq analysis apparatus, Illumina NextSeq500 (Illumina), it was found that the expression of mitochondria-related genes is significantly suppressed (data not shown) in Mipep KO mice compared to wild-type mice. From this, it was confirmed that mitochondrial biogenesis was suppressed.

### [Test Example 6]

In Test Example 6, a change in the amount of GDF15 protein in the plasma by knock-out of Mipep was checked. The change was checked by use of the Mouse/Rat GDF-15 Quantitative ELISA Kit (R&D Systems). The reaction was carried out in accordance with the protocol provided by the manufacturer.

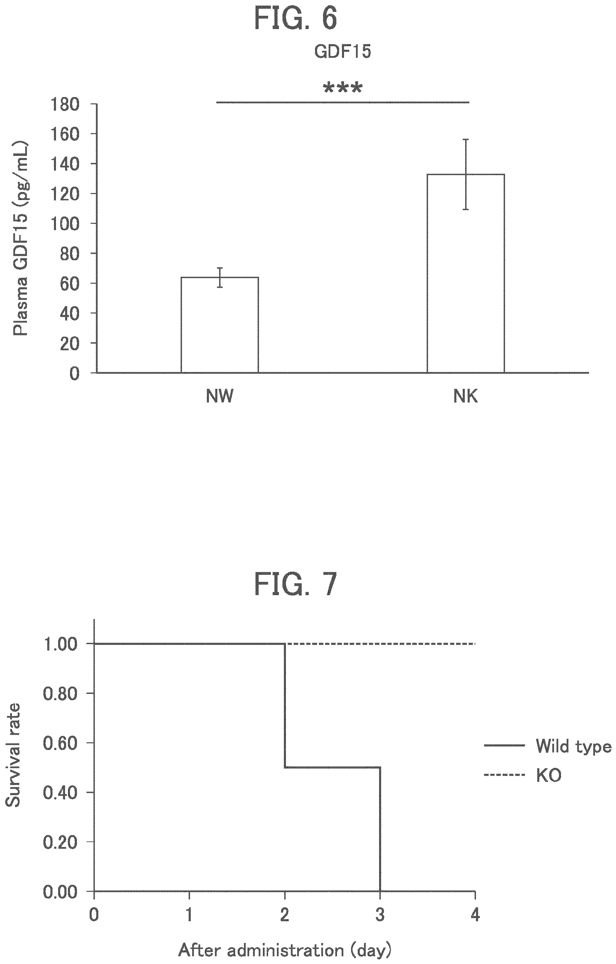

As shown in FIG. 6, the amount of GDF15 was high in the plasma of Mipep KO mice compared to the wild-type mice.

### [Test Example 7]

In Test Example 7, the effect of knock-out of Mipep on the survival after LPS was administered was checked. A group consisting of six male Mipep KO mice (40-45 weeks old) and a group of six wild-type mice (40-45 weeks old) were used. To each of the mice of both groups, LPS (Sigma-Aldrich) was intraperitoneally administered at a dose of 15 mg per body weight (1 kg). The number of survivors was counted every day after the administration and the survival rate was calculated based on the six mice as 100% and displayed as a survival curve.

As shown FIG. 7, resistance to LPS was significantly high in the Mipep KO mice compared to wild-type mice.

## Claims

1. A non-human animal or a part thereof having a totally or partially lost function of a Mipep gene in an adipose tissue or a lowered expression level of the Mipep gene in an adipose tissue compared to a wild type thereof.

2. Mesenchymal stem cells having a totally or partially lost function of a Mipep gene or a lowered expression level of the Mipep gene compared to a wild type thereof.

3. Adipocytes having a totally or partially lost function of a Mipep gene or a lowered expression level of the Mipep gene compared to a wild type thereof.

4. A therapeutic or prophylactic drug comprising at least one selected from the group consisting of mesenchymal stem cells or adipocytes having a totally or partially lost function of a Mipep gene or a lowered expression level of the Mipep gene compared to a wild type thereof, and a culture supernatant of the mesenchymal stem cells or adipocytes containing a mitokine.

5. The therapeutic or prophylactic drug according to claim 4, wherein the therapeutic or prophylactic drug is used for treating or preventing at least one selected from the group consisting of sepsis, ischemia/reperfusion injury, an inflammatory disease, a chronic kidney disease, obesity, atherosclerosis, a nonalcoholic fatty liver disease and other metabolic diseases.

6. A method for preparing a mitokine mixture, comprising collecting a plurality of types of mitokines from at least one selected from the group consisting of a non-human animal having a totally or partially lost function of a Mipep gene in an adipose tissue or a lowered expression level of the Mipep gene in an adipose tissue compared to a wild type thereof, and mesenchymal stem cells or adipocytes having a totally or partially lost function of the Mipep gene or a lowered expression level of the Mipep gene compared to a wild type thereof.
